# EUROPEAN PATENT APPLICATION

(11) **EP 4 085 921 A1**
(43) Date of publication of application: **09.11.2022**
(21) Application number: 21172100.6
(22) Date of filing: 04.05.2021
(51) Int. Cl.: A61K 38/08, A61P 31/14

(54) **ANGIOTENSIN-(1-7) IN THE TREATMENT OF SARS-COV RELATED DISEASES**

(71) Applicant: CU-Pharmaceuticals UG, 44263 Dortmund (DE)
(72) Inventor: SCHMITZ, Uwe, 32756 Detmold (DE)
(74) Representative: Michalski Hüttermann & Partner Patentanwälte mbB

(57) **Abstract**

The invention at hand relates to an Angiotensin-(1-7) (Asp-Arg-Val-Tyr-Ile-His-Pro) infusion for use in the treatment of coronavirus infection related diseases, wherein the Angiotensin-(1-7) is administered intravenously in the form of an aqueous solution comprising Angiotensin-(1-7) acetate at a concentration of larger or equal to 5 mcg/kg/24h and smaller or equal to 25 mcg/kg/24h. The invention further relates to an Angiotensin-(1-7) infusion.

## Description

The present invention relates to an Angiotensin-(1-7) (Asp-Arg-Val-Tyr-Ile-His-Pro) infusion for use in the treatment of coronavirus infection related diseases, wherein the Angiotensin-(1-7) is administered intravenously in the form of an aqueous solution comprising Angiotensin-(1-7) acetate at a concentration of larger or equal to 5 mcg/kg/24h and smaller or equal to 25 mcg/kg/24h. The invention further relates to an Angiotensin-(1-7) infusion.

SARS-CoV-2 is an abbreviation for severe acute respiratory syndrome coronavirus type 2, a new beta-coronavirus variant spreading since the beginning of 2020 all over the world. Beta-coronaviruses are enveloped, positive-stranded RNA viruses and this genus is known to infect mammals and birds. Usually, the clinical symptoms of diseases originating from these viruses in humans are more or less harmless, for instance including common cold like symptoms, but, nevertheless, as a function of the specific variant also severe and life-threatening illnesses like lung inflammation may be observed. Over the years several different virus variants were identified, wherein especially SARS-CoV-1 and the Middle-East respiratory syndrome (MERS)-CoV gained attention based on their exceptionally high mortality rates. The mortality was in most of the cases caused by respiratory failure and, therefore, the overall clinical classification Acute Respiratory Distress Syndrome (ARDS) emerged. Albeit the fact, that a lot of fundamental research was directed to the structure and biological function of coronaviruses in general and a vast amount of new evidences evolved on a very short timescale for the SARS-CoV-2 variant, this overall knowledge has not established a situation, wherein the physiopathology of the SARS-CoV-2 infection in humans is fully recognized and ARDS can be treated in all cases, appropriately.

As far as it is understood, SARS-CoV-1 and SARS-CoV-2 share the same biological target, i.e. the membrane bound angiotensin conversion enzyme 2 (ACE2) as a receptor for the binding domain of the virus spike proteins to enter host cells. High ACE2 densities are present in the respiratory tract, kidney, intestine, thyroid, vascular cells, heart muscle cells, adipose tissue cells and in other organs, explaining the vulnerability of these tissues to SARS-CoV infections. SARS-CoV induced ARDS starts with pulmonary symptoms with high deficit of blood oxygenation and indication of pneumonia and the worsening of the disease clearly indicates major impairment of the vascular endothelium, i.e., high blood pressure, thrombosis, pulmonary thromboembolism, stroke and myocardial infarct. In fact, diffuse pulmonary endothelial cell injury, that results in impairment of the alveolar-capillary barrier and increase in microvascular endothelial permeability, is considered central to the pathogenesis of acute respiratory distress syndrome.

The interaction of SARS-CoV and ACE2 is of course not only limited to the role of a door-opener for the virus. ACE2 is a very important regulatory enzyme of the renin-angiotensin system (RAS), controlling the cardiovascular system and the hydro-electrolyte balance. One central function is inter alia the conversion of the octapeptide Angiotensin 2 (Ang-2) to Angiotensin-(1-7) (Ang-(1-7)). ACE2 and other enzymes can form Ang-(1-7) via different routes, either directly or indirectly from the decapeptide Ang-1 or from the octapeptide Ang-2. Ang-2 binds to the angiotensin type 1 receptor (AT1R), a transmembrane G protein-coupled receptor, being present in a variety of cells. The activation of AT1R induces several physiological effects, wherein vascular permeability, vasoconstriction and pro-inflammatory cytokine release have to be mentioned. Based on the SARS-CoV/ACE2 interplay the functional equilibrium between the several constituents of the RAS-cascade are affected, resulting in an imbalance and contributing to the clinical symptoms and severity of ARDS.

The possibilities for the treatment of diseases based on the administration of RAS-constituents is also subject of several patent documents.

US 8,383,772 B2 discloses a peptide comprising the amino acid sequence of Asp¹-Arg²-Val³-Ser⁴-Ile⁵-His⁶-Cys⁷ and a pharmaceutical composition comprising the peptide and a pharmaceutically acceptable carrier.

Furthermore, EP 2 163 259 B1 discloses an Ang-(1-7) receptor agonist for use in the prevention and/or treatment of an acute lung injury in a subject.

EP 2 356 995 A2 teaches a pharmaceutical formulation comprising the peptide Angiotensin-(1-7) and/or its structural analogs, wherein the peptide is: (a) complexed with an alpha, beta or gamma hydrophilic cyclodextrin, or (b) incorporated into a biodegradable and biocompatible polymer, or (c) incorporated into a liposome and the use of said pharmaceutical formulation for the manufacture of a medicament for the treatment of arterial hypertension and its complications selected from the group of endothelial dysfunction, left ventricular hypertrophy, myocardial ischemia, stroke, hypertensive retinopathy, cardiovascular arteriosclerosis and heart failure in a mammal.

EP1 450 842 B1 for instance discloses a formulation comprising (a) D-Ala⁷-Angiotensin-(1-7) or D-Pro⁷-Angiotensin-(1-7) and (b) a cyclodextrin.

Despite the already existing treatment options and pharmaceutical formulations in the field of SARS-CoV infections, there is still an interest in new treatment regimes, that are able to improve the health status of hospitalized CoV-patients and, especially to reduce mortality of such patients.

This task is fulfilled by the use of an Angiotensin-(1-7) (Asp-Arg-Val-Tyr-Ile-His-Pro) infusion as defined in the independent claim. Preferred embodiments thereof are set forth in the dependent claims.

Above mentioned task is fulfilled by an Angiotensin-(1-7) (Asp-Arg-Val-Tyr-Ile-His-Pro) infusion for use in the treatment of coronavirus infection related diseases, wherein the Angiotensin-(1-7) is administered intravenously in the form of an aqueous solution comprising Angiotensin-(1-7) acetate at a concentration of larger or equal to 5 mcg/kg/24h and smaller or equal to 25 mcg/kg/24h.

Surprisingly, it has been found that Ang-(1-7) infusions in above defined concentration range can be used to reduce the severity of COVID and especially of SARS-CoV-2 related infections in patients already showing severe ARDS symptoms. Clinical data directed to the efficacy of said infusions revealed, that even in cases wherein the infusions are administered in the late-stages of SARS-CoV-2 infections, wherein the Angiotensin-(1-7) is administered to intensive care unit (ICU) patients diagnosed with severe pneumonia according to the WHO definitions, i.e., respiratory rate > 30 acts /minute, signs of respiratory effort, SaO₂< 90% in ambient air, the treatment is able to stabilize the patients and reduce the severeness of the ARDS symptoms. It was especially found, that the Angiotensin-(1-7) intravenous infusions are able to increase the amount of oxygen free days and reduce the mortality rate in this patient collective, compared to the standard care in the same clinical setting and considering the actual virus variant distribution in the clinical center.

While not wishing to be bound by theory, it is believed that the RAS receptors and their relationship play a crucial role in the pathogenesis of the disease caused by coronaviruses. As indicated above, ACE2 proteins serve as the cellular binding site for the spike proteins of SARS-CoV and especially SARS-CoV-2 and its binding leads to the virus entrance in the cells. Angiotensinogen, a protein primarily synthetized in the liver, is transformed into Angiotensin I (Ang I) by renin and is then cleaved into Angiotensin II (Ang II) by ACE. Ang II is then capable to trigger different responses in multiple tissues by binding to its specific receptors, primarily AT1 and AT2. Ang I could also be cleaved by ACE2 to produce Angiotensin-(1-9) (Ang-(1-9)), which can be further converted by ACE into Ang-(1-7) and this peptide can then bind to a specific receptor, called Mas-1 (MasR) and trigger a series of biological responses. The ACE2/Ang-(1-7)/MasR path appears to counterbalance the effects of the ACE/Ang II/AT1 axe, hence the system can be more precisely conceived as a wide and complex net of chemical and biological reactions, that can modulate a variety of biological processes, including inflammatory response, fibrogenesis, arterial tone and blood pressure, renal function, angiogenesis and endocrine functions. While the ACE/Ang II/AT1 path activation triggers an augmented blood pressure, increase in oxidative stress, inflammation, fibrosis, cytokine production, endothelial adhesion molecules, inflammatory cell migration, cell apoptosis and decrease the production of nitric oxide, the ACE2/Ang-(1-7)/MasR pathways regulate the opposite functions. Within the SARS-CoV-2 infection, it is postulated that ACE2 becomes severely downregulated, and this reduction in its enzymatic activity would contribute to an increase in Ang II, which cannot be transformed into Ang-(1-7), resulting in a profound imbalance of the RAS. Overall, the activation of the ACE2/Ang-(1-7)/MasR pathway should confer some cardiopulmonary protection. Ang-(1-7) mediated effects extend beyond the respiratory and cardiovascular system. It also has antithrombotic effects and a lower oxidative stress mediated by the adipose tissue endocrine function. This could be of importance in the context of a CoV-infection in general, but especially for SARS-CoV-2 induced respiratory failure and higher fatality rates observed in obese patients. Furthermore, the Angiotensin-(1-7) can oppose the increased activity of the Ang II/AT1 axis and counteract the decreased Ang-(1-7) levels/MasR expression. The administration of Ang-(1-7) could reestablish the right equilibrium and contribute to decrease pulmonary inflammation, consequently decreasing the disease symptoms. Most of the work and trials performed in this context concentrate on ACE or ACE2 and only little attention is given to the role of the final product in the cascade, i.e., Ang-(1-7), considered less likely to be efficient and difficult to be administered in the right concentration. The necessary Ang-(1-7) supplement should not only overcome the actual deficit, but should also act on the delicate equilibria of the complete enzymatic cascade, in order to improve the clinical situation of ARDS patients. According to our knowledge this is the first time, that clinical data be presented showing the efficacy of Ang-(1-7) administration. In addition, according to our best knowledge, it is the first time that clinical data are presented showing positive effect for ICU-patients showing high virus loads in an already late stage of infection. Furthermore, the positive effects of Angiotensin-(1-7) are very surprising, based on the fact that the Ang-(1-7) levels are not significantly increased upon the inventive use compared to a non-CoV control group.

The current invention relates to an Angiotensin-(1-7) (Asp-Arg-Val-Tyr-Ile-His-Pro) infusion for use in the treatment of CoV infection related diseases. Ang-(1-7) is a heptapeptide according to the above-mentioned sequence and this peptide or a pharmaceutically acceptable salt thereof is administered to the patients in a dissolved form. The Ang-(1-7) is administered in a liquid dosage form, an infusion. The infusion is used to treat symptoms or diseases originated from a CoV-infection. The CoV-infection may be any infection or co-infection, wherein coronaviruses are involved. The coronavirus may be any coronavirus-variant like SARS-CoV-1, SARS-CoV-2 or MERS and belongs to the family of coronaviridae, i.e. enveloped, positive-strand RNA viruses including the subfamilies Letovirinae and Orthocoronavirinae. The term virus variant includes the wild-type and possible mutations, as long as the virus belongs to the CoV-class. CoV can be identified based on an antigen-test, a PCR-test or based on test cultures. The CoV infection related diseases originate either directly or consequential from the contact and the interaction of the virus and human host cells. The interaction at least includes that a part of the viruses replicates in the host cells. Related diseases may for instance show the clinical symptoms of ARDS, pneumonia, reduced blood oxygen saturation etc..

The Angiotensin-(1-7) infusion is administered intravenously. The pharmaceutical dosage form for Ang-(1-7) administration is an infusion, wherein the dissolved drug is introduced into the bloodstream via a hollow needle and a syringe, which is pierced through the skin into the body.

The drug is injected into a patient's vein and an intramuscular or subcutaneous injection are out of scope of the invention at hand.

The infusion is present in the form of an aqueous solution comprising Angiotensin-(1-7) acetate. The drug is based on the angiotensin heptapeptide of the above defined amino-acid composition and the Ang-(1-7) is present as a function of the pH in a protonated form. The amino-acid is at least protonated once and the counterion is an aetate ion, i.e. CH₃COO⁻. The stoichiometry of the Ang-(1-7) and the acetate is at least 1:1, but also higher acetate contents may be possible. A suitable molar ratio (Ang-(1-7): Acetate) may for instance be in between 1:1 and 1:2.0, preferable between 1:1 and 1:1.5, more preferably between 1:1 and 1:1.2. This ratio may for instance influence the storage stability of the infusion.

The Angiotensin-(1-7) acetate is present in the solution corresponding to a concentration of larger or equal to 5 mcg/kg/24h and smaller or equal to 25 mcg/kg/24h. In order to achieve a significant clinical effect, it has been found useful to administer an Ang-(1-7) amount in above specified range. Within the clinical trial this amount has been found very safe without severe Ang-(1-7)-related side-effects. The administered concentration is calculated as a function of the patient's body-weight and the concentration in micro-grams (mcg) is administered in the course of 24 h or in shorter timescales, respectively. Lower concentrations may only result in an insufficient pharmacological effect, wherein higher concentrations may increase the risk of unwanted side-effects. It has to be mentioned that this dosage regimen can be combined with a wide variety of standard care treatments. It has to be outlined, that this dosage regimen can be also be used in combination with patients receiving complementary treatments with ACE-inhibitors or angiotensin receptor blockers (ARBs).

In a preferred embodiment of the use the Angiotensin-(1-7) infusion can be administered for a duration of at least 7 consecutive days. A treatment timescale of at least seven days has been found beneficial especially for patients already showing severe ARDS symptoms before the treatment start. Specifically, ICU patients can benefit from the minimum treatment duration. While not wishing to be bound by theory, it is believed that the duration is sufficient to establish a more physiological Angiotensin-(1-7) level in the blood stream and that within this timescale also the physiological level of the other members of the enzymatic cascade are brought to a more equilibrated level. Therefore, it is assumed that an active change of the enzymatic equilibria of the RAS-cascade is achieved, rather than just a supplement of diminished Angiotensin-(1-7) levels.

In another preferred embodiment of the use the Angiotensin-(1-7) infusion can be administered intravenously for at least 6 days at a concentration of larger or equal to 10 mcg/kg/24h and smaller or equal to 20 mcg/kg/24h. Especially, for the treatment of severely infected patients it has been found beneficial that Angiotensin-(1-7) in above defined concentration ranges and timescales is administered. These Angiotensin-(1-7) levels are able to supplement missing Angiotensin-(1-7) and, therefore, the negative physiological impact based on the And1-7 deficit is reduced. In addition, further influence on the overall RAS cascade can be expected, resulting in a faster recovery, higher blood oxygen saturation, lower need for invasive or non-invasive ventilation and a lower mortality. In a further preferred embodiment, the infusion can be administered intravenously for at least 6 days at a concentration of larger or equal to 12.5 mcg/kg/24h and smaller or equal to 18 mcg/kg/24h.

Within a further preferred aspect of the use the Angiotensin-(1-7) infusion can be administered at the first day at an Angiotensin-(1-7) concentration of larger or equal to 30 wt.-% and smaller or equal to 85 wt.-% with respect to the average administered Angiotensin-(1-7) concentration of the following days. For the treatment of hospitalized patients already showing severe symptoms of a CoV infection, it has been found beneficial that the Angiotensin-(1-7) administration is at least performed at two different Angiotensin-(1-7) levels, wherein the first concentration level is lower compared to the level in the following treatment time. While not wishing to be bound by theory, it is believed that better treatment result can be obtained by such levelling, because the organism is balanced by the first, lower Angiotensin-(1-7) infusion. In addition, it is assumed that the uptake and the effects of the higher doses in the consecutive phase is increased, based on the dose ramping. In a further preferred embodiment Angiotensin-(1-7) infusion can be administered at the first day at an Angiotensin-(1-7) concentration of larger or equal to 40 wt.-% and smaller or equal to 70 wt.-% and further preferred at the first day at an Angiotensin-(1-7) concentration of larger or equal to 45 wt.-% and smaller or equal to 65 wt.-% with respect to the average administered Angiotensin-(1-7) concentration of the following days.

Within a further preferred characteristic of the use the Angiotensin-(1-7) infusion can be administered on the first day at a concentration of larger or equal to 5 mcg/kg/24h and smaller to 10 mcg/kg/24h and administered on the following days in an average concentration of larger or equal to 10 mcg/kg/24h and smaller or equal to 25 mcg/kg/24h. For the treatment of hospitalized patients already showing severe CoV infection symptoms, it has been found beneficial that the Angiotensin-(1-7) administration is at least performed at two different Angiotensin-(1-7) levels, wherein the first concentration is significantly lower compared to the level in the remaining treatment time. While not wishing to be bound by theory, it is believed that better treatment results can be obtained by such levelling, because the organism is balanced by the first, lower Angiotensin-(1-7) infusion concentration. In addition, it is assumed that the uptake and the effects of the higher doses in the consecutive phase is increased, based on the dose ramping step. In a further preferred embodiment, an Angiotensin-(1-7) infusion can be administered at the first day at an Angiotensin-(1-7) concentration of larger or equal to 8.5 mcg/kg/24h and smaller or equal to 10 mcg/kg/24h and, further preferred, on the following days in an average concentration of larger or equal to 15 mcg/kg/24h and smaller or equal to 20 mcg/kg/24h.

In a further preferred embodiment of the use the Angiotensin-(1-7) infusion can be administered on the first day at a concentration of 5 mcg/kg/24h and administered on the following days in a concentration of 10 mcg/kg/24h. For the treatment of hospitalized patients already showing severe CoV infection symptoms it has been found beneficial that the Angiotensin-(1-7) administration is at least performed at two different Angiotensin-(1-7) levels, wherein the first concentration is lower compared to the level in the remaining treatment time. While not wishing to be bound by theory, it is believed that better treatment result can be obtained by such levelling, because the organism is balanced by the first, lower Angiotensin-(1-7) infusion concentration. In addition, it is assumed that the uptake and the effects of the higher doses in the consecutive phase is increased, based on the dose ramping step. For such dose ramping the beneficial effects of the clinical study has been demonstrated.

In another preferred embodiment of the use the infusion solution can be administered to hospitalized patients having a creatinine level of larger or equal to 0.5 mg/dL and smaller or equal to 2.0 mg/dL. Serum creatinine is an important indicator of kidney health, because it is an easily measured byproduct of muscle metabolism that is excreted unchanged by the kidneys. Creatinine itself is produced via a biological system involving creatine, phosphocreatine and adenosine triphosphate. Creatine is synthesized primarily in the liver from the methylation of glycocyamine by S-Adenosyl methionine. Serum creatinine is the most commonly used indicator of renal function. Elevated creatinine is not always representative of a true reduction in kidney function, but, nevertheless, in CoV related diseases it is a good indicator if the kidneys are also affected by the virus infection. Based on the treated patient collective, i.e. ICU patients showing clear signs of a CoV-infection, it has been found that also patients can be treated effectively already comprising an involvement of the kidneys. In a further preferred embodiment, the infusion can be administered to hospitalized patients having a creatinine level of larger or equal to 1.2 mg/dL and smaller or equal to 2.0 mg/dL and further preferred administered to hospitalized patients having a creatinine level of larger or equal to 1.5 mg/dL and smaller or equal to 2.0 mg/dL. Also, this group of ICU-patients can benefit from the Angiotensin-(1-7) treatment in the defined concentration range and it can be deduced from the clinical trial data that an Angiotensin-(1-7) infusion might help to effectively reduce creatinine levels.

In another preferred aspect of the use the infusion solution can be administered to hospitalized patients having a C-reactive protein-level of larger or equal to 100 mg/L. C-reactive protein (CRP) is a standard way to monitor the overall inflammatory state in hospitalized patients. CRP levels increase with every sort of infection (viral and bacterial), but also in case of injuries such as trauma, surgery, bleeding and so on. The data of the clinical trial reveal, that Angiotensin-(1-7) infusions are able to decrease the C-reactive protein-level upon infusion. While not wishing to be bound by theory, it is believed that the Angiotensin-(1-7) infusions are also able to show beneficial effects on in patients already showing a rather high level of inflammation.

In another preferred embodiment of the use the infusion solution can be administered to hospitalized patients having an Angiotensin-(1-7) level of larger or equal to 10.0 pg/mL and smaller or equal to 45 pg/mL. Especially, for such patient subgroup an Angiotensin-(1-7) infusion can result in a rather fast improvement of the clinical SARS-CoV- especially SARS-CoV-2-symptoms, like an improvement in fatigue, the recovery time for the smell or taste.

In another preferred aspect of the use the infusion solution can be administered to hospitalized patients having an Angiotensin-(1-5) level of larger or equal to 1.0 pg/mL and smaller or equal to 15 pg/mL. Angiotensin-(1-5) levels can be a marker of symptom severity in hospitalized CoV-patients. It has been found in the clinical trial, that Angiotensin-(1-7) can be helpful in patients showing such levels of Angiotensin-(1-5), wherein the infusion of Angiotensin-(1-7) result in a rather fast recovery and an increase of the Angiotensin-(1-5) level, respectively.

In another preferred characteristics of the use the infusion solution can be administered to hospitalized patients receiving invasive ventilation. The clinical trial data reveal, that Angiotensin-(1-7) infusions are very helpful in the context of patients receiving non-invasive or invasive ventilation. Ventilation, and here especially invasive ventilation, impact the inflammatory status of the patients, further driving the immune response away from a normal functioning. This patient subgroup can efficiently be treated with the inventive Angiotensin-(1-7) infusion. In the clinical trial it has been found that the days of supplemental oxygen can be reduced as a function of the Ang-(1-7) treatment.

In another preferred embodiment of the use the infusion solution can be administered to male hospitalized patients in an age group of larger or equal to 55 years and less or equal to 90 years. The clinical trial data reveal, that Angiotensin-(1-7) infusions are very helpful in the context of elderly patients, wherein it is principally difficult to treat elderly patients, based on the high likelihood of pre-existing factors complicating the treatment. The pre-existing factors can for instance be seen in a reduced immune response, obesity, diabetes or other illnesses prone to properly influence the body response to infections. This patient subgroup can efficiently be treated with the inventive Angiotensin-(1-7) infusion. In a further preferred embodiment, male hospitalized patients in an age group of larger or equal to 65 years and less or equal to 90 years can be treated in the inventive Ang-(1-7) concentration range.

In another preferred aspect of the use the infusion solution is administered to patients receiving an ACE inhibitor treatment. The clinical trial data reveal, that the Angiotensin-(1-7) infusion can also be combined with other standard care treatments. It is possible to treat also patients under ACE inhibitor medication.

A pharmaceutical Angiotensin-(1-7) infusion is also within the scope of the present invention, wherein the infusion comprises Angiotensin-(1-7) acetate and physiological saline solution. A rather fast physiological effect can be achieved by using an infusion solution comprising or consisting of physiological saline and Angiotensin-(1-7). The formulation is storage stable and based on the clean recipe no severe side-effects are expected upon intravenous administration. Such preferred composition can also show distinct advantages for the use of Angiotensin-(1-7) in the treatment of CoV-infections, especially SARS-CoV-2, wherein the Angiotensin-(1-7) is administered in the inventive dose regime.

In another preferred characteristics of the infusion the pH of the infusion solution can be larger or equal to pH 5.0 and smaller or equal to pH 7.5. For efficacy, bioavailability and storage stability it has been found useful, that the infusion solution is slightly acidic. Within above defined pH-range no negative effects were detected during the clinical trial. While not wishing to be bound by theory, it is assumed that the besides the storage stability effect also the physiological effects of the Angiotensin-(1-7) are influenced by the given formulation pH. In a further preferred embodiment, the infusion solution pH can be larger or equal to pH 5.05 and smaller or equal to pH 6.5, and further preferred larger or equal to pH 5.1 and smaller or equal to pH 6.0. Such preferred ranges can also show distinct advantages for the use of Angiotensin-(1-7) in the treatment of CoV-infections, especially SARS-CoV-2, wherein the Angiotensin-(1-7) is administered in the inventive dose regime.

Within a further preferred aspect of the infusion the molar ratio of the acetate and the Angiotensin-(1-7), calculated as acetate divided by Angiotensin-(1-7), can be larger or equal 1.0 and smaller or equal 2.0. It has been found that also the acetate amount in the Angiotensin-(1-7) acetate may influence the storage stability and the clinical efficacy. Within the given ratio storage stable and clinical effective infusions can be achieved. In a further preferred embodiment, the molar ratio can be larger or equal 1.0 and smaller or equal 1.5, and, further preferred, the ratio can be larger or equal 1.0 and smaller or equal 1.2. Such preferred ranges can also show distinct advantages for the use of Angiotensin-(1-7) in the treatment of CoV-infections, especially SARS-CoV-2, wherein the Angiotensin-(1-7) is administered in the inventive dose regime.

### Clinical Data

The following data are excerpts of a phase 0/1 clinical study registered under the UTN number: U1111-1255-7167 (Registro Brasileiro de Ensaios Clinicos). The study was conducted at the Eduardo de Menezes Hospital and Mater Dei Hospital in Belo Horizonte, Minas Gerais, Brazil.
1. Inclusion criteria: All patients were between 18 and 80 years old admitted to the ICU with a proved or suspected diagnosis of SARS-CoV-2 infection and a severe pneumonia diagnosis according to the WHO definition (clinical signs of pneumonia and one of the following criteria: respiratory rate > 30 acts /minute, signs of respiratory effort, SaO₂< 90% in ambient air) were assessed for eligibility.
2. Exclusion criteria: Patients with cancer (all stages) diagnosis, hemodynamic instability (need of vasopressors), pregnant women, immunocompromised patients, HIV positive patients, Limitations of care, cardiac failure as main cause of respiratory failure, idiopathic lung fibrosis, chronic Dialysis, decompensated liver cirrhosis, home oxygen therapy, inclusion in any other interventional trial.
3. Treatment: Patient in the phase I study received an intravenous administration of Ang-(1-7) at the initial dose of 5 mcg/kg/24h, augmented after 24 hours to 10 mcg/kg/24h for a maximum length of 7 days or until clinical improvement defined as discharge from ICU compared to placebo. The study drug was Angiotensin-(1-7) acetate and the drug is diluted in 250 mL of NaCl 0.9%. All patients received, in addition to the intervention, standard treatment according to guidelines of the Brazilian Ministry of Health for patients with SARS-CoV-2 disease (COVID-19). Management of any underlying comorbidity was at discretion of the attending physicians; the use of international guidelines for the monitoring and the adequate therapeutic interventions were recommended in all patients.
4. Main clinical outcomes of phase I: On average the SARS-CoV-2 patients exhibited 18 (SD +/- 8.9) oxygen free days by day 28 of the study. The mortality rate of SARS-CoV-2 patients admitted to ICU was 23.3 % (7/30). The average of the oxygen free days under Angiotensin-(1-7) treatment is higher compared to the average of oxygen free days of patients not receiving Angiotensin-(1-7) treatment. This qualitative outcome is based on the clinical experience gained with comparable SARS-CoV-2 patients (same time, same virus variant, same center). In addition, the mortality rate is significantly lower compared to SARS-CoV-2 infected, not Angiotensin-(1-7) treated ICU patients.

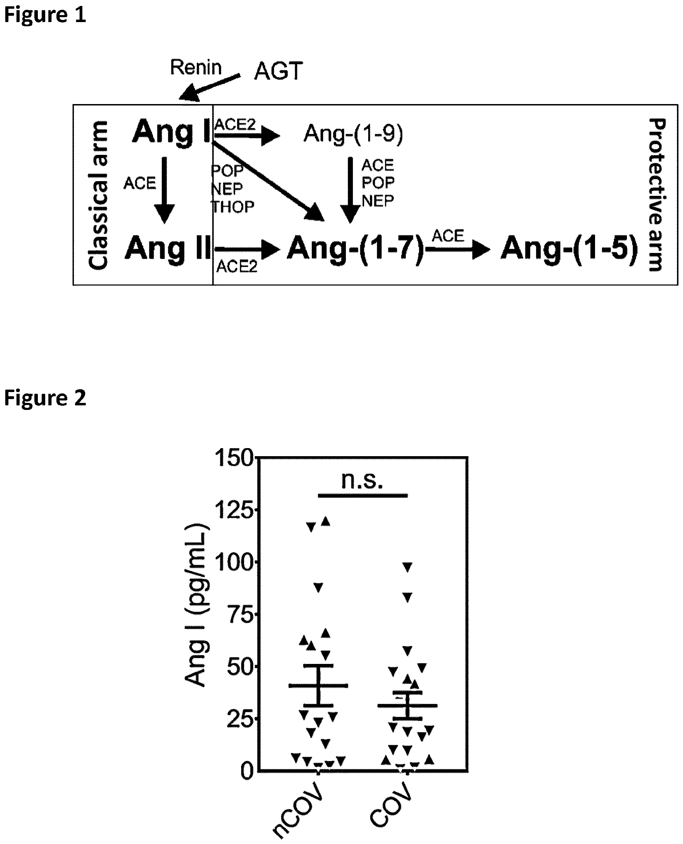

An overview of the interdependencies of some important components of the RAS-system is depicted in figure 1. The figure shows elements of the classic enzymatic cascade, the classic arm, comprising the conversion of Ang I by ACE to Ang II. The protective arm includes the conversion of Ang I by ACE2 to Ang-(1-9), wherein the latter is transformed via several mechanisms to Ang-(1-7). Ang-(1-7) is later on cleaved by ACE to Ang-(1-5).

In the clinical phase 0 the following study group was assessed:

| Phase 0 | non-COVID control | | COVID-19 patients | |
|---|---|---|---|---|
| | Man | Woman | Man | Woman |
| Group size | 15 | 4 | 14 | 5 |
| Age +/- (SEM) in years | 60 (6) | 42 (6) | 56 (3) | 49 (6) |
| ACEi | 3 | 0 | 3 | 0 |
| ARB | 3 | 0 | 4 | 4 |
| Outbreak +/- (SEM) in days | - | - | 11.2 (0.9) | 13.0 (0.7) |

The results of the phase 0 clinical study are displayed in the following figures. The figures show:
- Fig. 2: arterial blood concentration of the selected RAS peptides, in this case Ang I in the control (non-COVID) and the COVID-19-group including individuals under ACEi treatment;
- Fig. 3: arterial blood concentration of the selected RAS peptides, in this case Ang II in the control (non-COVID) and the COVID-19-group including individuals under ACEi treatment;
- Fig. 4: arterial blood concentration of the selected RAS peptides, in this case Ang-(1-7) in the control (non-COVID) and the COVID-19-group including individuals under ACEi treatment;
- Fig. 5: arterial blood concentration of the selected RAS peptides, in this case Ang-(1-5) in the control (non-COVID) and the COVID-19-group including individuals under ACEi treatment;
- Fig. 6: arterial blood concentration of the selected RAS peptides, in this case Ang I in the control (non-COVID) and the COVID-19-group excluding individuals under ACEi treatment;
- Fig. 7: arterial blood concentration of the selected RAS peptides, in this case Ang II in the control (non-COVID) and the COVID-19-group excluding individuals under ACEi treatment;
- Fig. 8: arterial blood concentration of the selected RAS peptides, in this case Ang-(1-7) in the control (non-COVID) and the COVID-19-group excluding individuals under ACEi treatment;
- Fig. 9: arterial blood concentration of the selected RAS peptides, in this case Ang-(1-5) in the control (non-COVID) and the COVID-19-group excluding individuals under ACEi treatment.

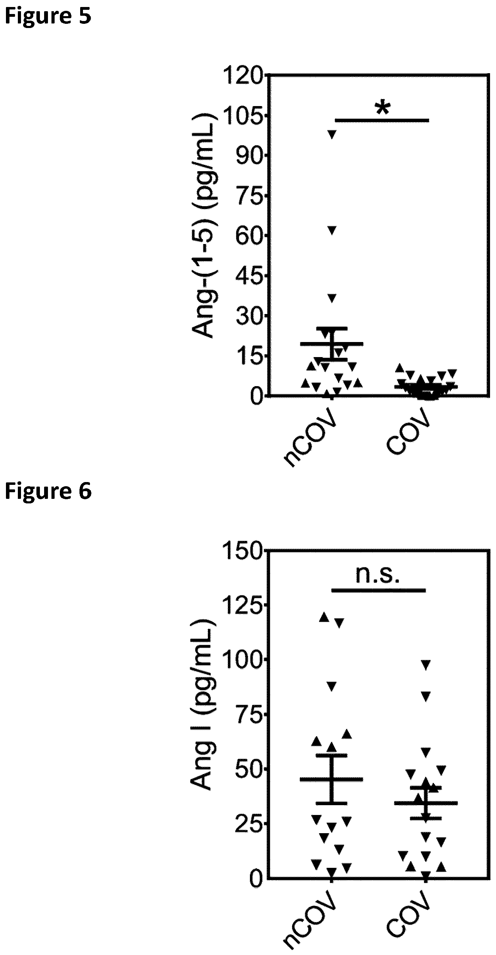

Figures 2-9 depict the arterial blood concentration of selected RAS peptides (Ang I, Ang II, Ang-(1-7) and Ang-(1-5)) from COVID-19 patients and non-COVID-19 volunteers. Figures 2-5 depict the result for all volunteers, wherein figures 6-9 show the result excluding individuals under ACE inhibitor (ACEi) treatment. Male volunteers are represented by (▼) and female volunteers by (A). Data are presented as mean ± standard deviation (SEM). A parametric t test was used for the statistical analysis.

Fig. 2 shows the arterial blood concentration of Ang I in the control (non-COVID) and the COVID-19-group including individuals under ACEi treatment. No statistical difference with respect to the Ang I concentration is visible in between both groups.

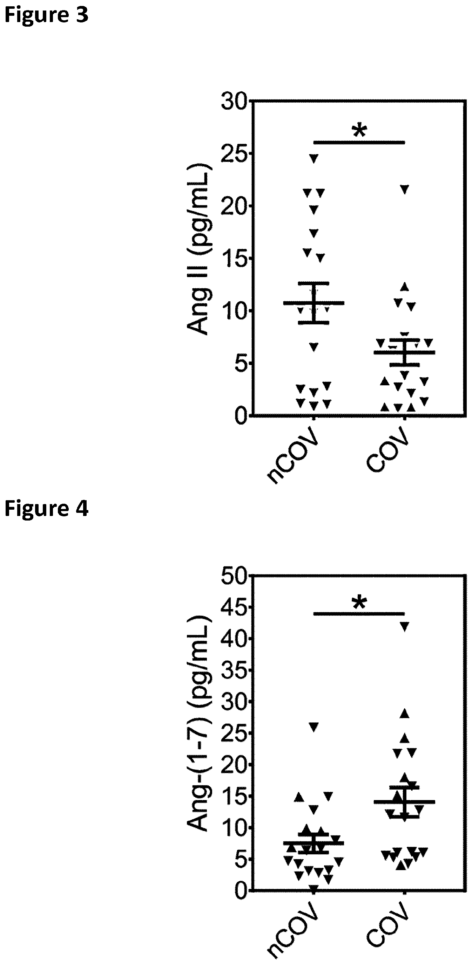

Fig. 3 shows the arterial blood concentration of Ang II in the control (non-COVID) and the COVID-19-group including individuals under ACEi treatment. In the non-COVID group the Ang II concentration is significantly higher compared to the COVID-19-group.

Fig. 4 shows the arterial blood concentration of Ang-(1-7) in the control (non-COVID) and the COVID-19-group including individuals under ACEi treatment. Interestingly, the Ang-(1-7) concentration is significantly higher in the COVID-group. This finding would discourage the skilled artisan to use Ang-(1-7) in order to improve the clinical situation of COVID-19 patients.

Fig. 5 shows the arterial blood concentration of Ang-(1-5) in the control (non-COVID) and the COVID-19-group including individuals under ACEi treatment. The Ang-(1-5) concentration is significantly lower in the COVID-19-group.

Fig. 6 shows the arterial blood concentration of Ang I in the control (non-COVID) and the COVID-19-group excluding individuals under ACEi treatment. No statistical difference with respect to the Ang I concentration is visible in between both groups.

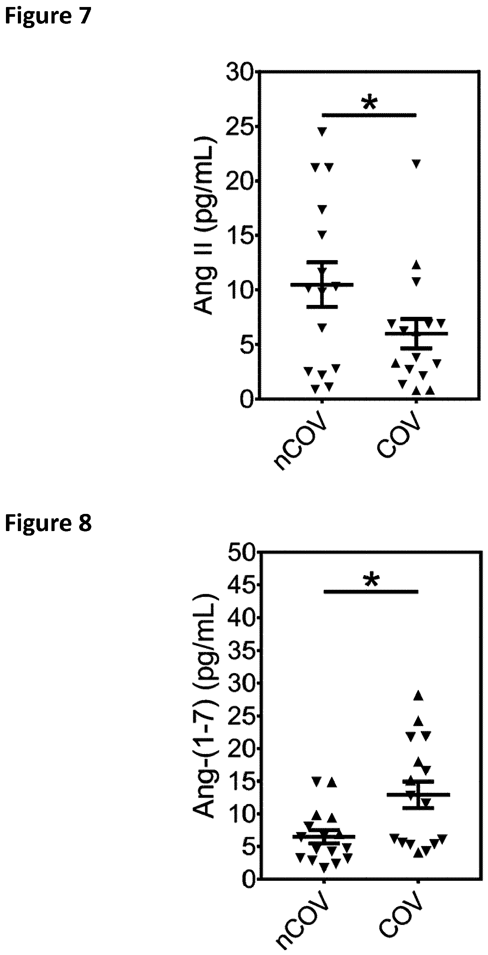

Fig. 7 shows the arterial blood concentration of Ang II in the control (non-COVID) and the COVID-19-group excluding individuals under ACEi treatment. In the non-COVID group the Ang II concentration is significantly higher compared to the COVID-19-group.

Fig. 8 shows the arterial blood concentration of Ang-(1-7) in the control (non-COVID) and the COVID-19-group excluding individuals under ACEi treatment. Interestingly, the Ang-(1-7) concentration is significantly higher in the COVID-19-group also in the case that patients receiving an ACEi treatment are excluded in the assessment. This finding would discourage the skilled artisan to use Ang-(1-7) in order to improve the clinical situation of a COVID-19 patient.

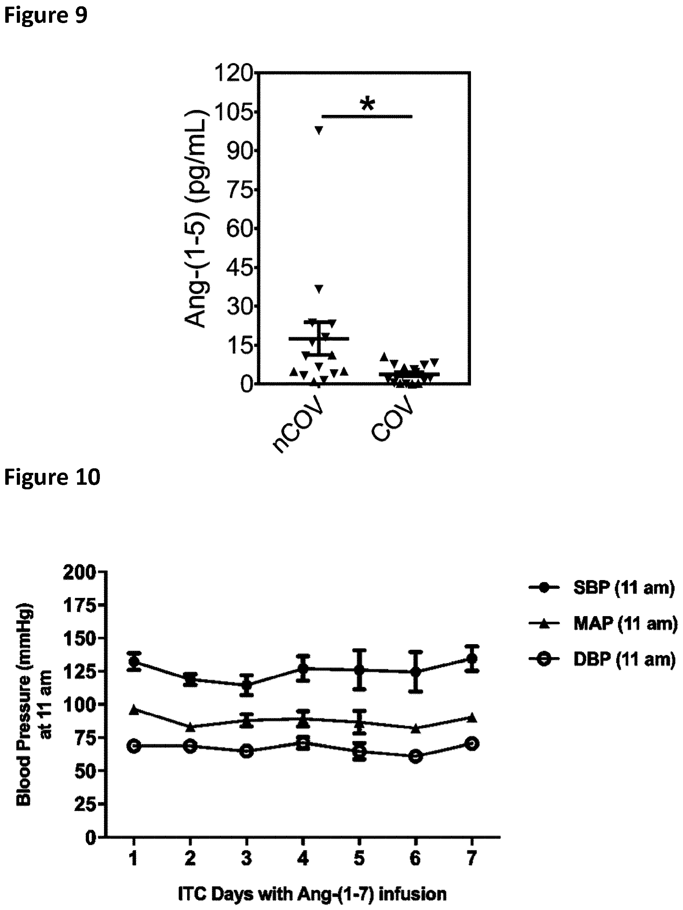

Fig. 9 shows the arterial blood concentration of Ang-(1-5) in the control (non-COVID) and the COVID-19-group excluding individuals under ACEi treatment. The Ang-(1-5) concentration is also significantly lower in the COVID-19-group excluding patients receiving an ACEi treatment.

The results of the phase I clinical study are displayed in the following figures. The figures display:
Fig. 10 the averaged blood pressure at 11 am as a function of the ICU days;

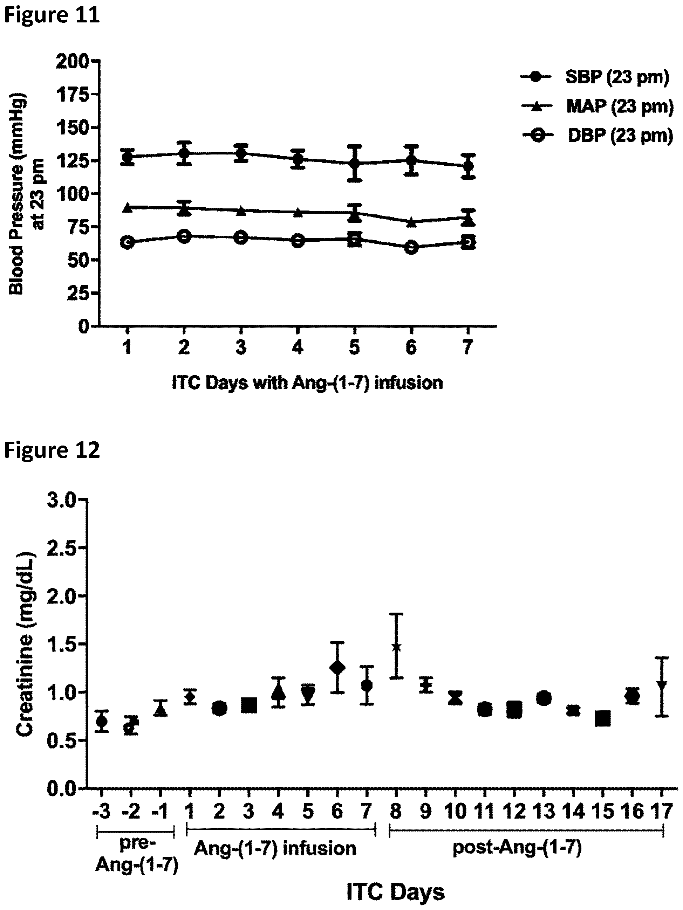

Fig. 11 the averaged blood pressure at 11 pm as a function of the ICU days;
Fig. 12 the averaged creatinine levels as a function of the ICU days;

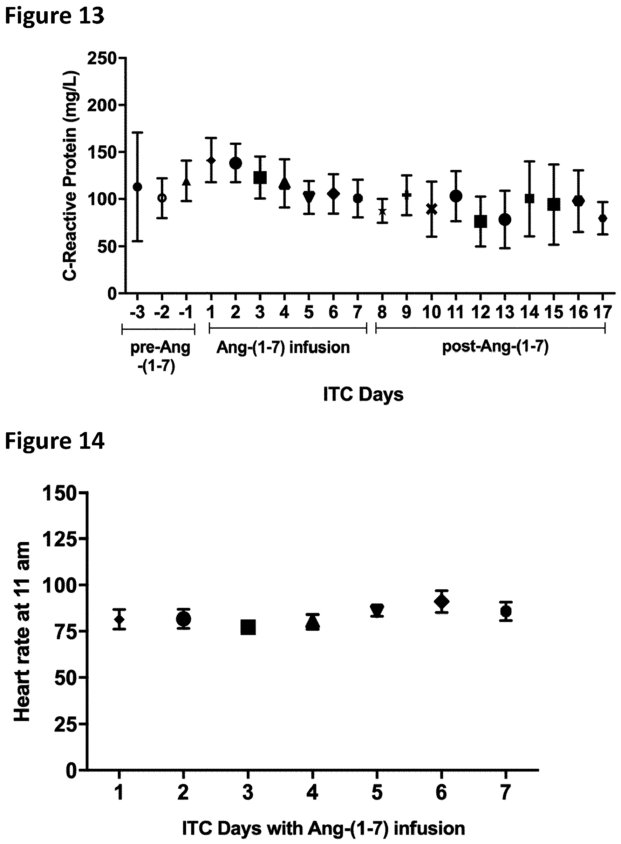

Fig. 13 the averaged C-reactive protein levels as a function of the ICU days;
Fig. 14 the averaged heart rate at 11 am as a function of the ICU days;

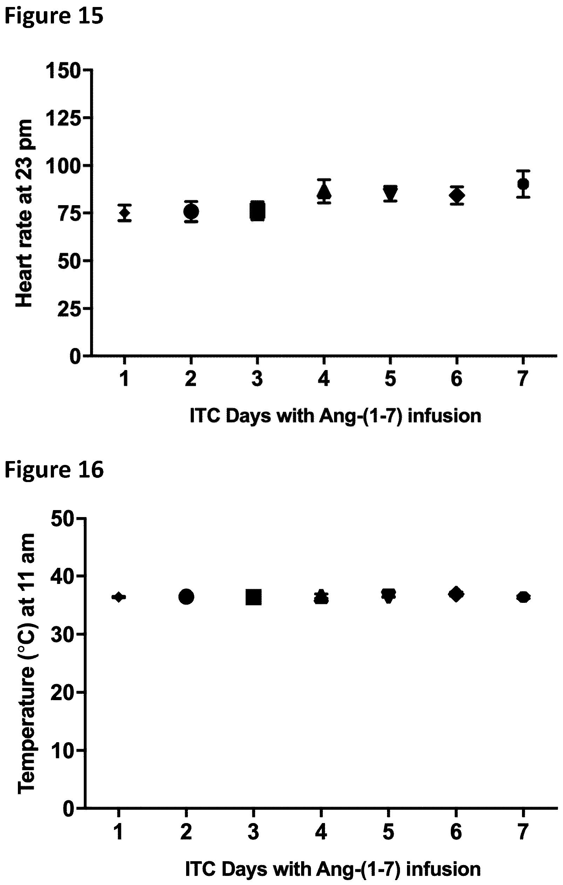

Fig. 15 the averaged heart rate at 11 pm as a function of the ICU days;
Fig. 16 the averaged temperature at 11 am as a function of the ICU days;

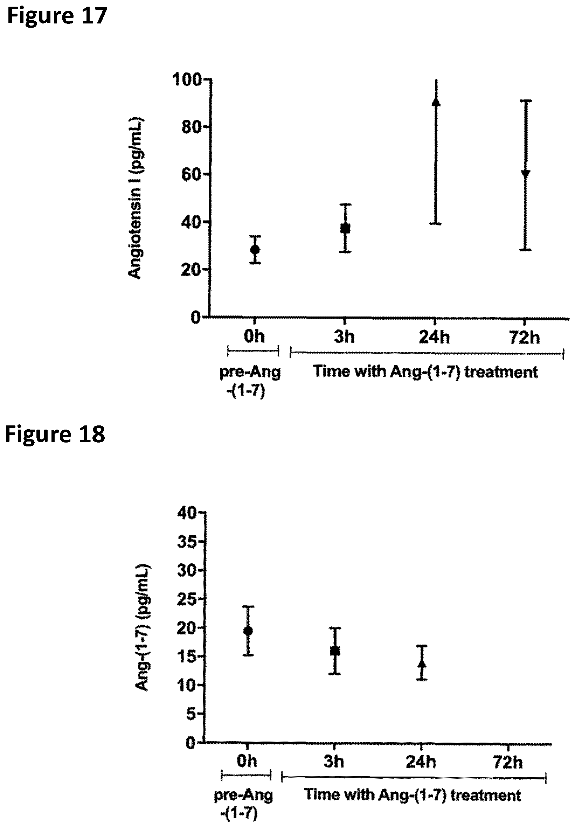

Fig. 17 the averaged Angiotensin I levels as a function of the treatment time interval;
Fig. 18 the averaged Angiotensin-(1-7) levels as a function of the treatment time interval;

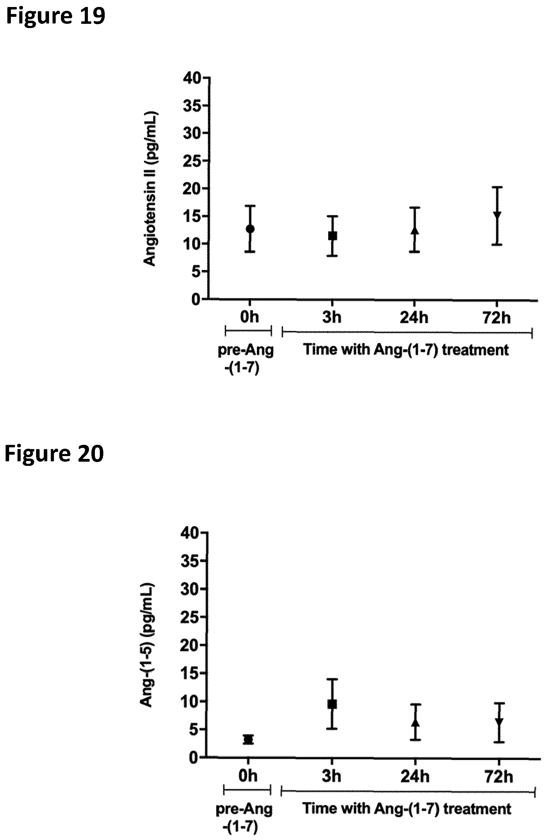

Fig. 19 the averaged Angiotensin II levels as a function of the treatment time interval;
Fig. 20 the averaged Angiotensin-(1-5) levels as a function of the treatment time interval.

Figures 10 and 11 display the averaged blood pressure as a function of ICU days with Ang-(1-7) infusion and assessment daytime for patients receiving the inventive amount of Ang-(1-7). The arterial pressure is an important parameter, because one expected adverse event after Ang-(1-7) infusion was hypotension. The abbreviations used in the graphs are SBP: Systolic blood pressure, MAP: Mean arterial pressure, DBP: Diastolic blood pressure and ITC: Intensive care unit. It can be deduced from the graphs that the blood pressure is not significantly influenced within the inventive Ang-(1-7) dose regime. Therefore, hypotension is not a side-effect of Ang-(1-7) administration in the defined concentration range.

Figure 12 displays the averaged creatinine levels as a function of the ICU days. The creatinine mean value at each timepoint in the COVID-19-cohort is displayed. Creatinine is a serum biomarker that is routinely measured as a reflection of the renal function. Normal values are considered to be lower than 1.2 mg/dL. Despite the real measurement of the glomerular filtration rate (GFR), the values are calculated by analyzing the fraction of plasmatic creatinine, urine creatinine and total volume of the 24h diuresis. The assessment of the creatinine value is important since it has been speculated that an Ang-(1-7) therapy could help to decrease the incidence of acute kidney injury (and thus renal failure). Despite the absence of a control group, it is difficult to show significant differences, nevertheless, the data suggest that the creatinine values are reduced upon Ang-(1-7) treatment.

Figure 13 displays the averaged C-reactive protein (CRP) blood concentrations of the COVID-19-cohort. CRP is a standard yet not very specific way to monitor the overall inflammatory state in hospitalized patients. The CRP-level increases with every sort of infection (viral and bacterial), but also after many different kinds of injuries such as trauma, surgery, bleeding and so on. These data must be interpreted in the light of the rate of superinfections, i.e. the patients enter the hospital with the COVID-19, but afterwards it is very likely that the patient develops many other infections, e.g. "ventilator associated pneumonia" or related to the necessity of other medical treatments. Nevertheless, this graph shows, that the overall inflammation is increasing in COVID-19 patients in the three days before the administration of the drug and decreased during the infusion. The overall inflammation seems also to decline post Ang-(1-7) administration.

Figures 14 and 15 display the averaged heart rate as a function of ICU days with Ang-(1-7) infusion and assessment daytime for patients receiving the inventive amount of Ang-(1-7). The graphs indicate that the heart rate is not influences by Ang-(1-7) administration.

Figure 16 displays the average temperatures of the COVID-19-cohort at 11 am during Ang-(1-7) infusion. It can be deduced from the graphs that the average temperatures are not significantly influenced by Ang-(1-7) administration.

Figure 17 displays the averaged Angiotensin I levels as a function of the treatment time interval. It can be deduced from the development of the Ang I concentrations that Ang-(1-7) administration seems to result in increased Ang I levels.

Figure 18 displays the averaged Angiotensin-(1-7) levels as a function of the treatment time interval. It can be deduced from the development of the Ang-(1-7) concentrations that Ang-(1-7) supplementation does not result in increased Ang-(1-7) levels. This fact indicates that other effects besides the sole Ang-(1-7) supplement are responsible for the positive effects of Ang-(1-7) administration.

Figure 19 displays the averaged Angiotensin II levels as a function of the treatment time interval. It can be deduced from the development of the Ang II levels that Ang-(1-7) supplementation seems to increase the Ang II levels.

Figure 20 displays the averaged Angiotensin 1-5 levels as a function of the treatment time interval. It can be deduced from the development of the Ang-(1-5) levels that Ang-(1-7) supplementation seems to increase the Ang-(1-5) concentration in the blood. This would be expected considering that Ang-(1-5) is an ACE-dependent product of Ang-(1-7). The formation of Ang-(1-5) may also explain the absence of gross changes in Ang-(1-7) following the infusion of the formulation.

## Claims

1. Angiotensin-(1-7) (Asp-Arg-Val-Tyr-Ile-His-Pro) infusion for use in the treatment of coronavirus infection related diseases, **characterized in that** the Angiotensin-(1-7) is administered intravenously in the form of an aqueous solution comprising Angiotensin-(1-7) acetate at a concentration of larger or equal to 5 mcg/kg/24h and smaller or equal to 25 mcg/kg/24h.

2. Use according to claim 1, wherein the Angiotensin-(1-7) infusion is administered for a duration of at least 7 consecutive days.

3. Use according to any one of the preceding claims, wherein the Angiotensin-(1-7) infusion is administered intravenously for at least 6 days at a concentration of larger or equal to 10 mcg/kg/24h and smaller or equal to 20 mcg/kg/24h.

4. Use according to any one of the preceding claims, wherein the Angiotensin-(1-7) infusion is administered at the first day at an Angiotensin-(1-7) concentration of larger or equal to 30 wt.-% and smaller or equal to 85 wt.-% with respect to the average administered Angiotensin-(1-7) concentration of the following days.

5. Use according to any one of the preceding claims, wherein the Angiotensin-(1-7) infusion is administered on the first day at a concentration of larger or equal to 5 mcg/kg/24h and smaller to 10 mcg/kg/24h and administered on the following days in an average concentration of larger or equal to 10 mcg/kg/24h and smaller or equal to 25 mcg/kg/24h.

6. Use according to any one of the preceding claims, wherein the Angiotensin-(1-7) infusion is administered on the first day at a concentration of 5 mcg/kg/24h and administered on the following days in a concentration of 10 mcg/kg/24h.

7. Use according to any one of the preceding claims, wherein the infusion solution is administered to hospitalized patients having a creatinine level of larger or equal to 0.5 mg/dL and smaller or equal to 2.0 mg/dL.

8. Use according to any one of the preceding claims, wherein the infusion solution is administered to hospitalized patients having a C-reactive protein-level of larger or equal to 100 mg/L.

9. Use according to any one of the preceding claims, wherein the infusion solution is administered to hospitalized patients having an Angiotensin-(1-7) level of larger or equal to 10.0 pg/mL and smaller or equal to 45 pg/mL.

10. Use according to any one of the preceding claims, wherein the infusion solution is administered to hospitalized patients having an Angiotensin 1-5 level of larger or equal to 1.0 pg/mL and smaller or equal to 15 pg/mL.

11. Use according to any one of the preceding claims, wherein the infusion solution is administered to hospitalized patients receiving invasive ventilation.

12. Use according to any one of the preceding claims, wherein the infusion solution is administered to male hospitalized patients in an age group of 55 to 90 years.

13. Use according to any one of the preceding claims, wherein the infusion solution is administered to patients receiving an ACE inhibitor treatment.

14. Pharmaceutical Angiotensin-(1-7) infusion, **characterized in that** the infusion comprises Angiotensin-(1-7) acetate and physiological saline solution.

15. Infusion according to claim 14, wherein the pH of the infusion is larger or equal to pH 5.0 and smaller or equal to pH 7.5.

16. Infusion according to claim 14 or claim 15, wherein the molar ratio of the acetate and the Angiotensin-(1-7), calculated as acetate divided by Angiotensin-(1-7), is larger or equal 1.0 and smaller or equal 2.0.
